# EUROPEAN PATENT SPECIFICATION

(11) **EP 2 730 303 B1**
(45) Date of publication and mention of the grant of the patent: **12.08.2015**
(21) Application number: 12007589.0
(22) Date of filing: 08.11.2012
(51) Int. Cl.: A61M 1/16

(54) **Albumin pump control in a MARS treatment apparatus**
Albuminpumpensteuerung in einer MARS-Behandlungsvorrichtung
Commande de pompe d'albumine dans un appareil de traitement MARS

(43) Date of publication of application: 14.05.2014
(73) Proprietor: Gambro Lundia AB, 220 10 Lund (SE)
(72) Inventor: Rada, Hiram, F-69008 Lyon (FR)
(74) Representative: Ponzellini, Gianmarco

(56) References cited:
- WO-A1-03/080154
- WO-A1-2011/131534
- US-A- 5 744 042

## Description

### DESCRIPTION

Apparatus for controlling movement of albumin solution in an extracorporeal blood treatment apparatus and associated methods are described herein.

### BACKGROUND

Extracorporeal blood treatment involves taking the blood from a patient, treating the blood outside the patient, and returning the treated blood to the patient. Extracorporeal blood treatment is typically used to extract undesirable matter or molecules from the patient's blood, and/or to add beneficial matter or molecules to the blood. The treatment is typically performed by sampling the patient's blood in a continuous flow, by introducing the blood into a primary chamber of a filter that is defined, at least in part, by a semi-permeable membrane. The semi-permeable membrane may selectively allow the unwanted matter contained in the blood pass through the membrane, from the primary chamber to the secondary chamber, and may selectively allow the beneficial matter contained in the liquid going into the secondary chamber pass through the membrane to the blood going into the primary chamber, according to the type of treatment.

Extracorporeal blood treatment is used with patients incapable of effectively eliminating matter from their blood. One example is a patient who is suffering from temporary or permanent kidney and/or liver failure. These and other patients may undergo extracorporeal blood treatment to add to or to eliminate matter from their blood, to maintain an acid-base balance, to eliminate excess body fluids, etc.

Although well-known and accepted techniques for removing water-soluble toxins from blood (to treat, e.g., end stage renal disease), the removal of protein-bound or lipophilic toxins is not as easily addressed. In particular, albumin-bound toxins (ABT) have been shown to be involved in the pathogenesis of different exogenous and endogenous intoxications in patients. ABTs such as butyric, valeric, caproic and caprylic acid, thyroxine and tryptophane, unconjugated bilirubin, mercaptans, digoxin-like immunoreactive substances, benzodiazepine-like substances, aromatic amino acids and phenols seem to be responsible for the induction of hepatic encephalopathy and cerebral edema in fulminant hepatic failure (FHF).

Approaches for removing both water-soluble toxins and protein-bound substances (PBS) from blood in extracorporeal treatment apparatus and methods are described in, e.g., U.S. Patent 5,744,042 (Stange et al.). Apparatus such as those described in U.S. Patent 5,744,042 are often referred to as Molecular Adsorbent Recirculating Systems (MARS) and involve removal of protein-bound substances through membrane transport using an albumin dialysate solution that is capable of removing both protein-bound substances (due to the presence of the albumin) and water-soluble toxins (normally removed using conventional dialysate solutions). Membranes that may be suitable for use in MARS are described in, e.g., US 5,744,042 as well as EP 2 380 610 A1 (Storr et al.; also published as WO 2011/131534 A1). WO 03/080154 A1 describes a method and a device to increase the efficiency of dialysis for the removal from blood of substances that are more or less tightly bound to carriers such as albumin. Here, this is accomplished by a simultaneous significant increase of the flow rate of the dialysis fluid and of the area of the membrane that separates the blood from the dialysis fluid, compared to conventional dialysis.

### SUMMARY

The extracorporeal blood treatment apparatus described herein involve control over flow of an albumin dialysate solution through an albumin solution circuit that is based on maintaining pressure of the albumin dialysate solution in the albumin circuit within a selected range.

Extracorporeal blood treatment apparatus as described herein include three different fluid circuits - a blood circuit in which blood is removed from and returned to a patient, an albumin solution circuit in which water-soluble toxins and PBS are removed from the blood using an albumin dialysate solution and in which the albumin dialysate solution is also regenerated, and a dialysate circuit in which water-soluble toxins are removed from the albumin dialysate solution. Because the apparatus described herein use both an albumin dialysate solution in the albumin solution circuit and a conventional dialysate in the dialysate circuit, the albumin dialysate solution will be referred to herein as the "albumin solution" to limit confusion with the dialysate used in the dialysate circuit.

Control over the flow of albumin solution in known MARS treatments typically matching the flow rate of the albumin solution with the extracorporeal blood flow rate. One potential disadvantage of such an approach is, however, that pressure in the albumin solution circuit is dependent on the pressure in the blood circuit. As a result, variation in blood flow through the blood circuit (due to, e.g., a kink in the tubing, a clot in a catheter, etc.) has a direct effect on the pressure in the albumin solution circuit. Variations in pressure of the albumin solution circuit can, in some instances, exceed the maximum allowed pressure which can require interrupting the MARS treatment until the pressure in the albumin solution circuit drops.

The extracorporeal blood treatment apparatus described herein involve controlling the flow rate in the albumin solution to hold the pressure in the albumin solution circuit within a selected range. In one or more embodiments, control over the pressure/flow rate within the albumin solution circuit may be independent of the pressure in the blood circuit. As a result, the maximum pressure limits in the albumin solution circuit will be rarely reached and interruptions in the MARS treatments may be reduced.

Although the albumin solution circuit flow rate is controlled based on pressure, the apparatus may further include secondary limits in flow rate control. For example, in one or more embodiments, the flow rate within the albumin solution circuit may be held at or below a selected maximum flow rate. Similarly, in one or more embodiments, the flow rate within the albumin solution circuit may be held at or above a selected minimum flow rate.

In one aspect, one or more embodiments of the apparatus configured to remove one or more substances from blood as described herein may include: a primary filter comprising a first semipermeable membrane separating a blood compartment from an albumin solution loading compartment; a blood circuit configured to move blood through the blood compartment of the primary filter using a blood pump; a dialysate filter comprising a second semipermeable membrane separating an albumin solution cleaning compartment from a dialysate compartment; a dialysate circuit configured to move dialysate through the dialysate compartment of the dialysate filter; and an albumin solution circuit configured to move albumin solution through the albumin solution loading compartment of the primary filter. The albumin solution circuit comprises: an albumin solution pump configured to pump the albumin solution into the albumin solution loading compartment of the primary filter; an albumin solution circuit pressure sensor configured to measure albumin circuit pressure in the albumin solution circuit; and a control unit operably connected to the albumin solution pump and the albumin solution circuit pressure sensor, wherein the control unit is configured to operate the albumin solution pump such that the albumin solution circuit pressure remains within a selected value range by varying an albumin solution flow rate in the albumin solution circuit.

In one or more embodiments of the apparatus configured to remove one or more substances from blood as described herein, the control unit is configured to control the albumin solution flow rate using the albumin solution pump independently of a blood flow rate provided by the blood pump in the blood circuit.

In one or more embodiments of the apparatus configured to remove one or more substances from blood as described herein, the control unit is configured to operate the albumin solution pump at or below a selected maximum flow rate.

In one or more embodiments of the apparatus configured to remove one or more substances from blood as described herein, the control unit is configured to operate the albumin solution pump at or above a selected minimum flow rate.

In one or more embodiments of the apparatus configured to remove one or more substances from blood as described herein, the albumin solution circuit pressure sensor is located in the albumin solution circuit downstream from the albumin solution pump and upstream from the albumin solution loading compartment of the primary filter.

In one or more embodiments of the apparatus configured to remove one or more substances from blood as described herein, the albumin solution circuit comprises one or more filters located between the albumin solution pump and the albumin solution loading compartment of the primary filter, wherein the one or more filters are configured to remove selected material from the fluid before the fluid enters the albumin solution loading compartment of the primary filter, and wherein the albumin solution circuit pressure sensor is located in the albumin solution circuit downstream from the albumin solution pump and upstream from the one or more filters.

In one or more embodiments of the apparatus configured to remove one or more substances from blood as described herein, the albumin solution pump is located downstream of the albumin solution cleaning compartment of the dialysate filter.

In one or more embodiments of the apparatus configured to remove one or more substances from blood as described herein, the albumin solution pump is located upstream of the albumin solution cleaning compartment of the dialysate filter.

In one or more embodiments of the apparatus configured to remove one or more substances from blood as described herein, changes in a blood pressure in the blood compartment of the primary filter changes albumin solution circuit pressure.

In one or more embodiments of the apparatus configured to remove one or more substances from blood as described herein, changes in a dialysate pressure in the dialysate compartment of the dialysate filter changes albumin solution circuit pressure.

In one or more embodiments of the apparatus configured to remove one or more substances from blood as described herein, the selected value range for the albumin solution circuit pressure comprises a selected constant value.

In a second aspect, described herein are one or more examples of a method of moving albumin solution through an albumin solution circuit in an extracorporeal blood treatment apparatus that includes a primary filter having a first semipermeable membrane separating a blood compartment from an albumin solution loading compartment, the albumin solution circuit configured to deliver albumin solution to the albumin solution loading compartment and to remove the albumin solution from the albumin solution loading compartment. The one or more examples of the method as described herein may include: pumping albumin solution through the albumin solution loading compartment of the primary filter at an albumin solution flow rate using an albumin solution pump; measuring albumin circuit pressure in the albumin solution circuit using an albumin solution circuit pressure sensor; and changing the albumin solution flow rate such that the albumin solution circuit pressure remains within a selected value range.

In one or more examples of the methods of moving albumin solution through an albumin solution circuit as described herein, the method may include limiting the albumin solution flow rate through the albumin solution loading compartment to a selected maximum flow rate or less.

In one or more examples of the methods of moving albumin solution through an albumin solution circuit as described herein, the method may include limiting the albumin solution flow rate through the albumin solution loading compartment to a selected minimum flow rate or higher.

In one or more examples of the methods of moving albumin solution through an albumin solution circuit as described herein, the method may include measuring the albumin solution circuit pressure downstream from the albumin solution pump and upstream from the albumin solution loading compartment of the primary filter.

In one or more examples of the methods of moving albumin solution through an albumin solution circuit as described herein, the method may include passing the albumin solution through one or more filters located between the albumin solution pump and the albumin solution loading compartment.

In one or more examples of the methods of moving albumin solution through an albumin solution circuit as described herein, the method may include measuring the albumin solution circuit pressure downstream from the albumin solution pump and upstream from the one or more filters, wherein the one or more filters are located between the albumin solution pump and the albumin solution loading compartment of the primary filter.

In one or more examples of the methods of moving albumin solution through an albumin solution circuit as described herein, the albumin solution circuit pressure changes in response to changes in pressure in the blood compartment of the primary filter.

In one or more examples of the methods of moving albumin solution through an albumin solution circuit as described herein, the extracorporeal blood treatment apparatus comprises a dialysate filter comprising a second semipermeable membrane separating an albumin solution cleaning compartment from a dialysate compartment, and the method may include pumping the albumin solution in the albumin solution circuit through the albumin solution cleaning compartment of the dialysate filter at the albumin solution flow rate using the albumin solution pump. In one or more embodiments, the albumin solution circuit pressure changes in response to changes in pressure in the dialysate compartment of the dialysate filter.

As used herein and in the appended claims, the singular forms "a," "an," and "the" include plural referents unless the context clearly dictates otherwise. Thus, for example, reference to "a" or "the" component may include one or more of the components and equivalents thereof known to those skilled in the art. Further, the term "and/or" means one or all of the listed elements or a combination of any two or more of the listed elements.

It is noted that the term "comprises" and variations thereof do not have a limiting meaning where these terms appear in the accompanying description. Moreover, "a," "an," "the," "at least one, and "one or more" are used interchangeably herein.

The above summary is not intended to describe each embodiment or every implementation of the extracorporeal blood treatment apparatus described herein. Rather, a more complete understanding of the invention will become apparent and appreciated by reference to the following Description of Illustrative Embodiments and claims in view of the accompanying figures of the drawing.

### BRIEF DESCRIPTION OF THE VIEWS OF THE DRAWING

FIG. 1 depicts one illustrative embodiment of an extracorporeal blood treatment apparatus as described herein.
FIG. 2 is a block diagram of a control unit and components operably connected to it.
FIG. 3 depicts another illustrative embodiment of an extracorporeal blood treatment apparatus as described herein.

### DESCRIPTION OF ILLUSTRATIVE EMBODIMENTS

In the following description of illustrative embodiments, reference is made to the accompanying figures of the drawing which form a part hereof, and in which are shown, by way of illustration, specific embodiments. It is to be understood that other embodiments may be utilized and structural changes may be made without departing from the scope of the present invention.

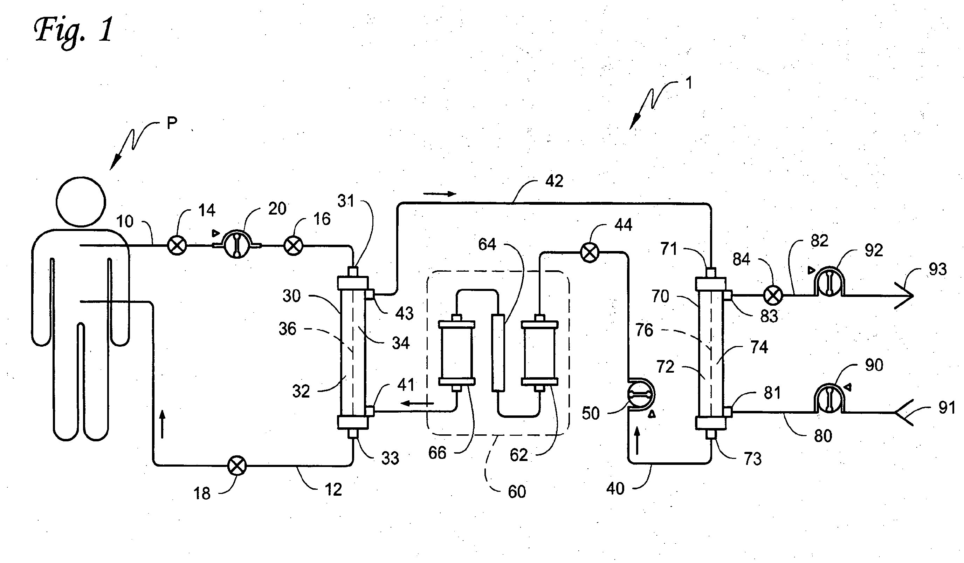

In the illustrative embodiment depicted in FIG. 1, the extracorporeal blood treatment apparatus 1 includes a blood circuit having an arterial line 10 that delivers blood from a patient P to a blood compartment 32 of a primary filter 30. The blood in the blood circuit is returned to the patient P through a venous return line 12. The blood compartment 32 of the primary filter 30 is separated from an albumin solution loading compartment 34 in the primary filter 30 by a semipermeable membrane 36. Although the primary filter 30 is depicted simplistically as having only one blood compartment 32 and one albumin solution loading compartment 34, the primary filter 30 depicted in FIG. 1 should not be construed to limit the apparatus described herein to sue albumin solution circuit h a simple embodiment. For example, the blood compartments or the albumin solution loading compartments in the primary filters of extracorporeal blood treatment apparatus described herein may be, e.g., defined by a plurality of hollow fibers constructed of a semipermeable material as is known in the art.

As the patient's blood moves through the blood compartment 32 along the membrane 36, water-soluble and protein-bound toxins in the blood are transported through the membrane 36 and into the albumin solution in the albumin solution loading compartment 34 (such that the albumin solution is "loaded" with the water-soluble and protein-bound toxins in the blood). The membrane 36 is impermeable to albumin and preferably to other valuable proteins (e.g., hormones, clotting factors, etc.) so that the albumin remains in the albumin solution and the valuable proteins remain in the blood.

The blood circuit of the apparatus 1 of FIG. 1 also includes a blood pump 20 that is configured to move blood through the blood compartment 32 of the primary filter 30, with the blood entering the blood compartment 32 through an inlet 31 to which the arterial line 10 is connected. Blood in the blood circuit leaves the blood compartment 32 through an outlet 33 to which the venous line 12 is connected. Although depicted as a roller pump, the blood pump 20 may be of any suitable design (e.g., a roller pump, piston pump, diaphragm pump, etc.) or other flow control mechanism (e.g., valves, clamps, etc.), etc.

The blood circuit depicted in FIG. 1 also includes optional pressure sensors configured to measure pressure at various locations in the blood circuit. In the depicted embodiment, the blood circuit includes an access pressure sensor 14 located between the patient P and the blood pump 20. The access pressure sensor 14 may be used to monitor pressure in the arterial line 10 downstream of the patient P and upstream of the blood pump 20. The blood circuit of FIG. 1 also includes a filter pressure sensor 16 located downstream from the blood pump 20 and upstream of the blood chamber 32 of the primary filter 30. The filter pressure sensor 16 can be used to monitor pressure in the arterial line 10. A third pressure sensor in the form of a return pressure sensor 18 is located along the venous return line 12 downstream of the blood compartment 32 of the primary filter 30 and upstream of the patient P. The return pressure sensor 18 monitors pressure in the blood circuit after the blood has passed through the blood compartment 32 and before it is returned to the patient P.

The extracorporeal blood treatment apparatus 1 depicted in FIG. 1 also includes an albumin solution circuit configured to move albumin solution through the albumin solution loading compartment 34 of the primary filter 30. In the albumin solution circuit, the albumin solution enters the albumin solution loading compartment 34 of the primary filter through an inlet 41 and leaves the albumin solution loading compartment 34 through an outlet 43.

The albumin solution circuit also includes an albumin solution cleaning compartment 72 in a dialysate filter 70 that also includes a dialysate compartment 74 that forms a part of the dialysate circuit. The albumin solution cleaning compartment 72 of the dialysate filter 70 is separated from a dialysate compartment 74 in the dialysate filter 70 by a semipermeable membrane 76. Although the dialysate filter 70 is depicted simplistically as having only one albumin solution cleaning compartment 72 and one dialysate compartment 74, the dialysate filter 70 may be, e.g., constructed of semipermeable hollow fibers as is known in the art and the simplistic depiction of FIG. 1 should not be construed to limit the apparatus described herein to such a simple embodiment.

Albumin solution leaving the albumin solution loading compartment 34 through outlet 43 travels through fluid line 42 to inlet 71 where the albumin solution enters the albumin solution cleaning compartment 72 of dialysate filter 70. The albumin solution leaves the albumin solution cleaning compartment 72 through an outlet 73 to which fluid line 40 is attached. The fluid line 40 extends to the inlet 41 of the albumin solution loading compartment 34 such that albumin solution leaving the albumin solution cleaning compartment 72 is delivered to the albumin solution loading compartment 34 after passing through other elements of the albumin solution circuit as discussed herein.

For example, the albumin solution circuit in the apparatus 1 depicted in FIG. 1 includes an albumin solution pump 50 configured to move albumin solution through the albumin circuit. In the embodiment depicted in FIG. 1, the albumin solution pump 50 is located along fluid line 40 downstream of the outlet 73 of the albumin solution cleaning compartment 72. Although depicted as a roller pump, the albumin solution pump 50 may be of any suitable design (e.g., a roller pump, piston pump, diaphragm pump, etc.) or other flow control mechanism (e.g., valves, clamps, etc.), etc.

In the apparatus depicted in FIG. 1, the albumin solution circuit includes an albumin solution regeneration unit 60 positioned along fluid line 40 that includes an activated carbon adsorber 62, a particulate filter 64, and an anion exchanger 66. Albumin solution passing through the fluid line 40 from the albumin solution cleaning compartment 72 of dialysate filter 70 to the albumin solution loading compartment 34 of the primary filter 30 passes through the activated carbon adsorber 60 after which the albumin solution passes through particulate filter 64 to remove any carbon particulates picked up in the activated carbon adsorber 62. After passing through the particulate filter 64, the albumin solution passes through the anion exchanger 66 before being delivered to the albumin solution loading compartment 34.

Albumin solution moving through the albumin circuit is recycled or cleaned after it picks up both water-soluble toxins and protein-bound substances from the blood moving through the blood circuit in the albumin solution loading compartment 34 of the primary filter 30.

In particular, dialysate delivered into the dialysate compartment 74 of the dialysate filter 70 provides clean dialysate that is used to remove a therapeutically effective amount of water-soluble toxins from the albumin solution passing through the albumin solution cleaning compartment 72. As the albumin solution, loaded with water-soluble toxins as well as protein-bound substances, moves through the albumin solution cleaning compartment 72 along the membrane 76, water-soluble toxins in the albumin solution are transported through the membrane 76 and into the dialysate in the dialysate compartment 74 (such that the albumin solution is "cleaned" of the water-soluble toxins picked up by the albumin solution in the primary filter 30). The membrane 76 in the dialysate filter 70 is impermeable to the albumin and the protein-bound toxins carried by the albumin so that the albumin and the protein-bound substances remain in the albumin solution after the albumin solution passes through the albumin solution cleaning compartment 72 of the dialysate filter.

A therapeutically effective amount of the protein-bound substances in the albumin solution leaving the albumin solution cleaning compartment 72 are removed from the albumin solution by the albumin solution regeneration unit 60. In particular, the albumin in the albumin solution is reconditioned or regenerated such that it can again bind to protein-bound substances in blood in the albumin solution loading compartment 34 of the primary filter.

The albumin circuit of the apparatus depicted in FIG. 1 also includes an albumin solution circuit pressure sensor 44 that, in the depicted embodiment, is positioned along fluid line 40 upstream of the activated carbon adsorber 60 and the anion exchanger 64. The albumin solution circuit pressure sensor 44 is also positioned downstream of both the albumin solution cleaning compartment 72 and the albumin solution pump 50.

Due to its position, the albumin solution circuit pressure sensor 44 is capable of monitoring the pressure in the albumin circuit at the inlet to the albumin solution regeneration unit 60. In the depicted embodiment, the inlet to the albumin solution regeneration unit 60 is the inlet to the activated carbon adsorber 62 because the adsorber 62 is upstream of the particulate filter 64 and the anion exchanger 66.

The illustrative embodiment of an extracorporeal blood treatment apparatus depicted in FIG. 1 also includes a dialysate circuit. The dialysate circuit is used to provide dialysate to the dialysate compartment 74 of the dialysate filter 70. The dialysate in the dialysate compartment 74 is used to remove water-soluble toxins from the albumin solution moving through the albumin solution cleaning compartment 72 of the dialysate filter 70 as described herein.

Dialysate is delivered to the dialysate compartment 74 of the dialysate filter 70 through a delivery line 80 that is connected to inlet 81 on the dialysate filter 70. A dialysate pump 90 is used to deliver the dialysate from a dialysate source 91 to the dialysate compartment 74 of the dialysate filter 70. The dialysate source 91 may be in any suitable form, e.g., fixed volume containers (e.g., bags, etc.).

The dialysate delivered into the dialysate compartment 74 of the dialysate filter 70 leaves the dialysate compartment 74 through an outlet 83. The outlet 83 is connected to a drain line 82 through which the used dialysate is delivered to a drain 93. In the depicted embodiment, the dialysate circuit includes an effluent pump 92 that is used to assist in removing the used dialysate from the dialysate compartment 74 of the dialysate filter 70.

Although the dialysate pump 90 and the optional effluent pump 92 are both depicted as roller pumps, the pumps may be of any suitable design (e.g., a roller pump, piston pump, diaphragm pump, etc.) or other flow control mechanism (e.g., valves, clamps, etc.), etc.

The dialysate circuit of the apparatus depicted in FIG. 1 also includes a dialysate circuit pressure sensor 84 that, in the depicted embodiment, is positioned along effluent line 82 upstream of the effluent pump 92 and downstream of the dialysate compartment 74 of the dialysate filter 70.

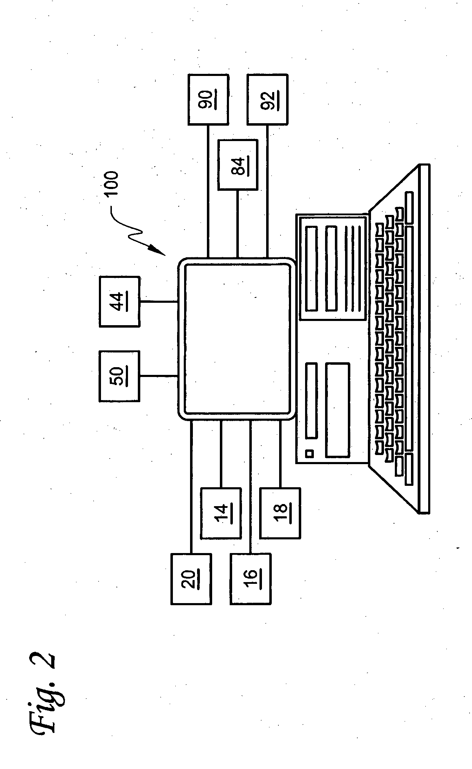

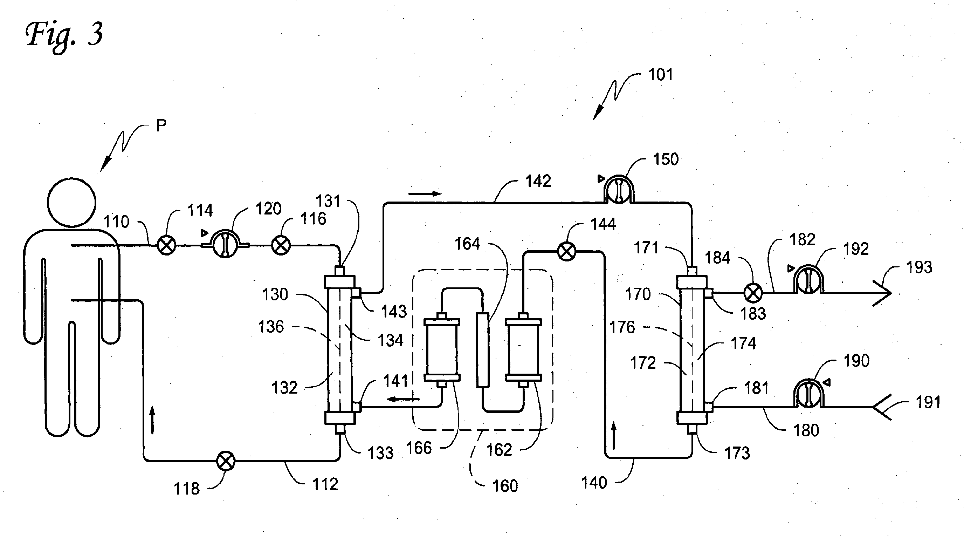

The extracorporeal blood treatment apparatus described herein includes a control unit that is not depicted in FIG. 1 (or in connection with the alternative illustrative embodiment depicted in FIG. 3). FIG. 2 is, however, a block diagram depicting various components of the extracorporeal blood treatment apparatus described herein that may be operably connected to the control unit 100. In the illustrative embodiment depicted in FIG. 2, the access pressure sensor 14, filter pressure sensors 16, and return pressure sensor 18 of the blood circuit are all operably connected to the control unit 100. In addition, the blood pump 20 of the blood circuit is also operably connected to the control unit 100. In the albumin circuit, the inlet pressure sensor 44 and the albumin pump 50 are also both operably connected to the control unit 100. In the dialysate circuit, the dialysate pump 90, effluent pump 92 and effluent pressure sensor 84 are all operably connected to the control unit 100.

The control unit 100 may be provided in any suitable form and may, for example, include memory and a controller. The controller may, for example, be in the form of one or more microprocessors, Application Specific Integrated Circuit (ASIC) state machines, etc. The control unit 100 may include one or more of any suitable input devices configured to allow a user to operate the apparatus (e.g., keyboards, touchscreens, mice, trackballs, etc.), as well as display devices configured to convey information to a user (e.g., monitors (which may or may not be touchscreens), indicator lights, etc.).

The methods of moving albumin solution through an albumin solution circuit in an extracorporeal blood treatment apparatus will be described herein with respect to the extracorporeal blood treatment apparatus 1 depicted in FIG. 1, although the methods described herein may be practiced with any apparatus capable of performing the methods described herein. With reference to FIG. 1, the methods described herein include moving albumin solution through an albumin solution loading compartment 34 of a primary filter 30 at an albumin solution flow rate using an albumin solution pump 50. The primary filter 30 includes a blood compartment 32 separated from the albumin solution loading compartment 34. The method further includes measuring albumin circuit pressure in the albumin solution circuit using an albumin solution circuit pressure sensor 44 and changing the albumin solution flow rate such that the albumin solution circuit pressure remains within a selected value range.

In one or more examples, the selected value range for the albumin solution circuit pressure may be a single selected value, i.e., the range may be a single setpoint, while in one or more other examples, the selected value range for the albumin solution circuit pressure may include an upper limit and a lower limit that are two different values. In one or more examples, an example of a possible upper limit may be, e.g., 500 mmHg. In one or more examples, a possible lower limit may be, e.g., 300 mmHg. The actual values for the upper and lower pressure limits may, in one or more examples, depend on other factors such as, e.g., blood return pressure in the blood circuit, albumin pump flow rates, etc.

One or more examples of the methods of moving albumin solution through the albumin solution circuit as described herein may include limiting the albumin solution flow rate through the albumin solution loading compartment 34 to a selected maximum flow rate or less. In other words, even if the albumin solution circuit pressure as measured by, e.g., the albumin solution circuit pressure sensor 44 is below the selected value range, the albumin solution flow rate may not be increased if the albumin solution flow rate is already at a selected maximum flow rate. In one or more examples, the selected maximum flow rate may be selected based on a maximum flow rate that can be safely delivered using the albumin solution pump 50 in the albumin solution circuit.

One or more examples of the methods of moving albumin solution through the albumin solution circuit as described herein may include controlling the albumin solution flow rate through the albumin solution loading compartment 34 to a selected minimum flow rate or higher. In other words, even if the albumin solution circuit pressure as measured by, e.g., the albumin solution circuit pressure sensor 44 is above the selected value range, the albumin solution flow rate may be held at a selected minimum flow rate if the albumin solution flow rate is already at that minimum value. In one or more examples, the selected minimum flow rate may be based on a minimum flow rate required to remove a sufficient amount of water-soluble toxins and protein-bound substances from blood in the blood compartment 32 of the primary filter 30 (across from the albumin solution in the albumin solution loading compartment 34 of the primary filter 30).

In one or more examples, the method of moving albumin solution through the albumin solution circuit as described herein may include measuring the albumin solution circuit pressure downstream from the albumin solution pump 50 and upstream from the albumin solution loading compartment 34 of the primary filter 30.

In one or more examples, the method of moving albumin solution through the albumin solution circuit as described herein may include passing the albumin solution through a regeneration unit 60 to remove protein-bound substances from the albumin solution and/or otherwise restore the ability of the albumin in the albumin solution to remove protein-bound substances from blood, with the regeneration unit 60 being located between the albumin solution pump 50 and the albumin solution loading compartment 34.

In one or more examples that use such a regeneration unit, the methods described herein may include measuring the albumin solution circuit pressure downstream from the albumin solution pump 50 and upstream from the regeneration unit 60, wherein the regeneration unit 60 is located between the albumin solution pump 50 and the albumin solution loading compartment 34 of the primary filter 30.

In one or more examples, the methods of moving albumin solution through the albumin solution circuit as described herein may involve changes in the albumin solution circuit pressure in response to changes in pressure of blood in the blood compartment 32 of the primary filter 30. Such changes will, however, be taken into account by the control methods described herein.

In one or more examples, the methods of moving albumin solution through the albumin solution circuit as described herein may include pumping the albumin solution in the albumin solution circuit through the albumin solution cleaning compartment 72 of the dialysate filter 70 at the albumin solution flow rate using the albumin solution pump 50. In such a method, the extracorporeal blood treatment apparatus includes a dialysate filter 70 having a semipermeable membrane separating the albumin solution cleaning compartment 72 from the dialysate compartment 74. Water-soluble toxins are removed from the albumin solution moving through the albumin solution cleaning compartment 72 and transferred to dialysate that is in the dialysate compartment 72 across the semipermeable membrane. In one or more examples of the methods described herein, the albumin solution circuit pressure changes in response to changes in pressure in the dialysate compartment 74 of the dialysate filter 70. Such changes will, however, be taken into account by the control methods described herein.

An alternative illustrative embodiment of an extracorporeal blood treatment apparatus 101 is depicted in FIG. 3. The extracorporeal blood treatment apparatus 101 depicted in FIG. 3 includes a blood circuit, a dialysate circuit, and an albumin solution circuit located between the blood circuit and the dialysate circuit similar to the arrangement in the extracorporeal blood circuit of FIG. 1.

The blood circuit includes an arterial line 110 that delivers blood from a patient P to a blood compartment 132 of a primary filter 130. The blood in the blood circuit is returned to the patient P through a venous line 112. The blood compartment 132 is separated from an albumin solution loading compartment 134 in the primary filter 130 by a semipermeable membrane 136. Although the primary filter 130 is depicted simplistically as having only one blood compartment 132 and one albumin solution loading compartment 134, the simplistic depiction of FIG. 3 should not be construed to limit the apparatus described herein to such a simple embodiment.

The blood circuit of the apparatus 101 of FIG. 3 also includes a blood pump 120 that is configured to move blood through the blood compartment 132, with the blood entering the blood compartment 132 through an inlet 131 to which the arterial line 110 is connected. Blood in the blood circuit leaves the blood compartment 132 through an outlet 133 to which the venous line 112 is connected. Although depicted as a roller pump, the blood pump 120 may be of any suitable design (e.g., a roller pump, piston pump, diaphragm pump, etc.) or other flow control mechanism (e.g., valves, clamps, etc.), etc.

The blood circuit depicted in FIG. 3 also includes optional pressure sensors configured to measure pressure at various locations in the blood circuit. In the depicted embodiment, the blood circuit includes an access pressure sensor 114 located between the patient P and the blood pump 110, a filter pressure sensor 116 located downstream from the blood pump 120 and upstream of the blood chamber 132, and a return pressure sensor 118 located along the venous return line 112 downstream of the blood compartment 132 and upstream of the patient P.

The albumin solution circuit in the extracorporeal blood treatment apparatus 101 depicted in FIG. 3 is also configured to move albumin solution through the albumin solution loading compartment 134 of the primary filter 130. In the albumin solution circuit, the albumin solution enters the albumin solution loading compartment 134 through an inlet 141 and leaves the albumin solution loading compartment 134 through an outlet 143. The albumin solution circuit also delivers albumin solution to an albumin solution cleaning compartment 172 of a dialysate filter 170 that has a dialysate compartment 174 that forms a part of the dialysate circuit as discussed above in connection with the illustrative embodiment of FIG. 1.

Albumin solution leaving the albumin solution loading compartment 134 through outlet 143 travels through fluid line 142 to inlet 171 where the albumin solution enters the albumin solution cleaning compartment 172. The albumin solution leaves the albumin solution cleaning compartment 172 through an outlet 173 to which fluid line 140 is attached. The fluid line 140 extends to the inlet 141 of the albumin solution loading compartment 134 such that albumin solution leaving the albumin solution cleaning compartment 172 is delivered to the albumin solution loading compartment 134 after passing through other elements of the albumin solution circuit as discussed herein.

The albumin solution circuit in the apparatus depicted in FIG. 3 includes an albumin solution pump 150 configured to move albumin solution through the albumin circuit. In the embodiment depicted in FIG. 3, the albumin solution pump 150 is located along fluid line 142 upstream of the inlet 171 of the albumin solution cleaning compartment 172. Although depicted as a roller pump, the albumin solution pump 150 may be of any suitable design (e.g., a roller pump, piston pump, diaphragm pump, etc.) or other flow control mechanism (e.g., valves, clamps, etc.), etc.

In the apparatus depicted in FIG. 3, the albumin solution circuit includes, as depicted in connection with the embodiment of FIG. 1, an albumin solution regeneration unit 160 positioned along fluid line 140 that includes an activated carbon adsorber 162, a particulate filter 164, and an anion exchanger 166.

Albumin solution moving through the albumin circuit is recycled or cleaned after it picks up both water-soluble toxins and protein-bound substances from the blood moving through the blood circuit in the albumin solution loading compartment 134 of the primary filter 130. In particular, dialysate delivered into the dialysate compartment 174 provides clean dialysate that is used to remove a therapeutically effective amount of water-soluble toxins from the albumin solution passing through the albumin solution cleaning compartment 172 as discussed above. As is also discussed above, a therapeutically effective amount of the protein-bound substances in the albumin solution leaving the albumin solution cleaning compartment 172 are removed from the albumin solution by the albumin solution regeneration unit 160.

The albumin circuit of the apparatus depicted in FIG. 3 also includes an albumin solution circuit pressure sensor 144 that, in the depicted embodiment, is position along fluid line 140 upstream of the activated carbon adsorber 160 and the anion exchanger 164. The albumin solution circuit pressure sensor 144 is also positioned downstream of both the albumin solution cleaning compartment 172 and the albumin solution pump 150 in the embodiment depicted in FIG. 3. Alternatively, the albumin solution circuit pressure sensor 144 could be positioned downstream of the albumin solution pump 150 and upstream of the albumin solution cleaning compartment 172 (i.e., between the albumin solution pump 150 and the albumin solution cleaning compartment 172) in one or more embodiments.

Due to its position, the albumin solution circuit pressure sensor 144 is capable of monitoring the pressure in the albumin circuit at the inlet to the albumin solution regeneration unit 160. In the depicted embodiment, the inlet to the albumin solution regeneration unit 160 is the inlet to the activated carbon adsorber 162 because the adsorber 162 is upstream of the particulate filter 164 and the anion exchanger 166.

The illustrative embodiment of an extracorporeal blood treatment apparatus depicted in FIG. 3 also includes a dialysate circuit similar to that in the embodiment depicted in FIG. 1. The dialysate circuit includes a dialysate compartment 174 in the dialysate filter 170. The dialysate in the dialysate compartment 174 is used to remove water-soluble toxins from the albumin solution moving through the albumin solution cleaning compartment 172.

Dialysate is delivered to the dialysate compartment 174 through a delivery line 180 that is connected to inlet 181 on the dialysate filter 170. A dialysate pump 190 is used to deliver the dialysate from a dialysate source 191 to the dialysate compartment 174. The dialysate source 191 may be in any suitable form, e.g., fixed volume containers (e.g., bags, etc.). The dialysate delivered into the dialysate compartment 174 leaves the dialysate compartment 174 through an outlet 183. The outlet 183 is connected to a drain line 182 through which the used dialysate is delivered to a drain 193. In the depicted embodiment, the dialysate circuit includes an effluent pump 192 that is used to assist in removing the used dialysate from the dialysate compartment 174.

Although the dialysate pump 190 and the optional effluent pump 192 are both depicted as roller pumps, the pumps may be of any suitable design (e.g., a roller pump, piston pump, diaphragm pump, etc.) or other flow control mechanism (e.g., valves, clamps, etc.), etc.

The dialysate circuit of the apparatus depicted in FIG. 3 also includes a dialysate circuit pressure sensor 184 that, in the depicted embodiment, is positioned along effluent line 182 upstream of the effluent pump 192 and downstream of the dialysate compartment 174.

The complete disclosure of the patents, patent documents, and publications identified herein are incorporated by reference in their entirety as if each were individually incorporated.

Illustrative embodiments of the blood treatment apparatus and methods of using the same are discussed and reference has been made to possible variations.

## Claims

1. An apparatus (1) configured to remove one or more substances from blood, wherein the apparatus comprises:
a primary filter (30) comprising a first semipermeable membrane (36) separating a blood compartment (32) from an albumin solution loading compartment (34);
a blood circuit configured to move blood through the blood compartment (32) of the primary filter (30) using a blood pump (20);
a dialysate filter (70) comprising a second semipermeable membrane (76) separating an albumin solution cleaning compartment (72) from a dialysate compartment (74);
a dialysate circuit configured to move dialysate through the dialysate compartment (74) of the dialysate filter (70);
an albumin solution circuit configured to move albumin solution through the albumin solution loading compartment (34) of the primary filter (30), wherein the albumin solution circuit comprises:
an albumin solution pump (50) configured to pump the albumin solution into the albumin solution loading compartment (34) of the primary filter (30);
an albumin solution circuit pressure sensor (44) configured to measure albumin circuit pressure in the albumin solution circuit; and
a control unit (100) operably connected to the albumin solution pump (50) and the albumin solution circuit pressure sensor (44), wherein the control unit (100) is configured to operate the albumin solution pump (50) such that the albumin solution circuit pressure remains within a selected value range by varying an albumin solution flow rate in the albumin solution circuit.

2. An apparatus (1) according to claim 1, wherein the control unit (100) is configured to control the albumin solution flow rate using the albumin solution pump (50) independently of a blood flow rate provided by the blood pump (20) in the blood circuit.

3. An apparatus (1) according to any one of claims 1 to 2, wherein the control unit (100) is configured to operate the albumin solution pump (50) at or below a selected maximum flow rate.

4. An apparatus (1) according to any one of claims 1 to 3, wherein the control unit (100) is configured to operate the albumin solution pump (50) at or above a selected minimum flow rate.

5. An apparatus (1) according to any one of claims 1 to 4, wherein the albumin solution circuit pressure sensor (44) is located in the albumin solution circuit downstream from the albumin solution pump (50) and upstream from the albumin solution loading compartment (34) of the primary filter (30).

6. An apparatus (1) according to any one of claims 1 to 4, wherein the albumin solution circuit comprises one or more filters (62, 64, 66) located between the albumin solution pump (50) and the albumin solution loading compartment (34) of the primary filter (30), wherein the one or more filters (62, 64, 66) are configured to remove selected material from the fluid before the fluid enters the albumin solution loading compartment (34) of the primary filter (30), and wherein the albumin solution circuit pressure sensor (44) is located in the albumin solution circuit downstream from the albumin solution pump (50) and upstream from the one or more filters.

7. An apparatus (1) according to any one of claims 1 to 6, wherein the albumin solution pump (50) is located downstream of the albumin solution cleaning compartment (72) of the dialysate filter (70).

8. An apparatus (1) according to any one of claims 1 to 6, wherein the albumin solution pump (50) is located upstream of the albumin solution cleaning compartment (72) of the dialysate filter (70).

9. An apparatus (1) according to any one of claims 1 to 8, wherein changes in a blood pressure in the blood compartment (32) of the primary filter (30) change albumin solution circuit pressure.

10. An apparatus (1) according to any one of claims 1 to 9, wherein changes in a dialysate pressure in the dialysate compartment (74) of the dialysate filter (70) change albumin solution circuit pressure.

11. An apparatus (1) according to any one of claims 1 to 10, wherein the selected value range for the albumin solution circuit pressure comprises a selected constant value.

## Patentansprüche

1. Vorrichtung (1) konfiguriert zum Entfernen eines oder mehrerer Stoffe aus Blut, wobei die Vorrichtung umfasst:
einen Primärfilter (30) umfassend eine erste semi-permeable Membran (36), die ein Blutabteil (32) von einem Albuminlösungsladeabteil (34) trennt;
einen Blutkreislauf konfiguriert zum Bewegen von Blut durch das Blutabteil (32) des Primärfilters (30) unter Verwendung einer Blutpumpe (20);
einen Dialysatfilter (70) umfassend eine zweite semipermeable Membran (76), die ein Albuminlösungsspülabteil (72) von einem Dialysatabteil (74) trennt;
einen Dialysatkreislauf konfiguriert zum Bewegen von Dialysat durch das Dialysatabteil (74) des Dialysatfilters (70);
einen Albuminlösungskreislauf konfiguriert zum Bewegen der Albuminlösung durch das Albuminlösungsladeabteil (34) des Primärfilters (30), wobei der Albuminlösungskreislauf umfasst:
eine Albuminlösungspumpe (50) konfiguriert zum Pumpen der Albuminlösung in das Albuminlösungsladeabteil (34) des Primärfilters (30);
einen Albuminlösungskreislaufdrucksensor (44) konfiguriert zum Messen des Albuminkreislaufdruckes im Albuminlösungskreislauf; und
eine Steuereinheit (100), die mit der Albuminlösungspumpe (50) und dem Albuminlösungskreislaufdrucksensor (44) operativ verbunden ist, wobei die Steuereinheit (100) konfiguriert ist zum Betreiben der Albuminlösungspumpe (50), so dass der Albuminlösungskreislaufdruck durch Ändern der Albuminlösungsflussrate im Albuminlösungskreislauf innerhalb eines ausgewählten Wertebereiches bleibt.

2. Vorrichtung (1) nach Anspruch 1, wobei die Steuereinheit (100) konfiguriert ist zum Steuern der Albuminlösungsflussrate unter Verwendung der Albuminlösungspumpe (50), unabhängig von einer Blutflussrate, die von der Blutpumpe (20) im Blutkreislauf bereitgestellt wird.

3. Vorrichtung (1) nach einem der Ansprüche 1 bis 2, wobei die Steuereinheit (100) konfiguriert ist zum Betreiben der Albuminlösungspumpe (50) bei oder unterhalb einer ausgewählten Maximalflussrate.

4. Vorrichtung (1) nach einem der Ansprüche 1 bis 3, wobei die Steuereinheit (100) konfiguriert ist zum Betrieben der Albuminlösungspumpe (50) bei oder oberhalb einer ausgewählten Minimalflussrate.

5. Vorrichtung (1) nach einem der Ansprüche 1 bis 4, wobei sich der Albuminlösungskreislaufdrucksensor (44) im Albuminlösungskreislauf abwärts von der Albuminlösungspumpe (50) und aufwärts vom Albuminlösungsladeabteil (34) des Primärfilters (30) befindet.

6. Vorrichtung (1) nach einem der Ansprüche 1 bis 4, wobei der Albuminlösungskreislauf einen oder mehreren Filter (62, 64, 66) umfasst, die sich zwischen der Albuminlösungspumpe (50) und dem Albuminlösungsladeabteil (34) des Primärfilters (30) befinden, wobei der eine oder die mehreren Filter (62, 64, 66) konfiguriert sind zum Entfernen ausgewählten Materials aus dem Fluid, bevor das Fluid in das Albuminlösungsladeabteil (34) des Primärfilters (30) gelangt, und wobei sich der Albuminlösungskreislaufdrucksensor (44) im Albuminlösungskreislauf abwärts von der Albuminlösungspumpe (50) und aufwärts vom einen oder von mehreren Filtern befindet.

7. Vorrichtung (1) nach einem der Ansprüche 1 bis 6, wobei sich die Albuminlösungspumpe (50) abwärts vom Albuminlösungsspülabteil (72) des Dialysatfilters (70) befindet.

8. Vorrichtung (1) nach einem der Ansprüche 1 bis 6, wobei sich die Albuminlösungspumpe (50) aufwärts vom Albuminlösungsspülabteil (72) des Dialysatfilters (70) befindet.

9. Vorrichtung (1) nach einem der Ansprüche 1 bis 8, wobei Änderungen eines Blutdruckes im Blutabteil (32) des Primärfilters (30) den Albuminlösungskreislaufdruck verändern.

10. Vorrichtung (1) nach einem der Ansprüche 1 bis 9, wobei Änderungen eines Dialysatdruckes im Dialysatabteil (74) des Dialysatfilters (70) den Albuminlösungskreislaufdruck verändern.

11. Vorrichtung (1) nach einem der Ansprüche 1 bis 10, wobei der ausgewählte Wertbereich für den Albuminlösungskreislaufdruck einen ausgewählten konstanten Wert umfasst.

## Revendications

1. Appareil (1) configuré pour éliminer une ou plusieurs substances du sang, où l'appareil comprend:
un filtre primaire (30) comprenant une première membrane semi-perméable (36) séparant un compartiment du sang (32) d'un compartiment de chargement de la solution d'albumine (34);
un circuit du sang configuré pour déplacer le sang à travers le compartiment du sang (32) du filtre primaire (30) en utilisant une pompe du sang (20) ;
un filtre du dialysat (70) comprenant une deuxième membrane semi-perméable (76) séparant un compartiment de rinçage de la solution d'albumine (72) d'un compartiment du dialysat (74);
un circuit du dialysat configuré pour déplacer le dialysat à travers le compartiment du dialysat (72) du filtre du dialysat (70);
un circuit de la solution d'albumine configuré pour déplacer la solution d'albumine à travers le compartiment de chargement de la solution d'albumine (34) du filtre primaire (30), où le circuit de la solution d'albumine comprend:
une pompe de la solution d'albumine (50) configurée pour pomper la solution d'albumine dans le compartiment de chargement de la solution d'albumine (34) du filtre primaire (30);
un capteur de pression du circuit de la solution d'albumine (44) configuré pour mesurer la pression du circuit d'albumine dans le circuit de la solution d'albumine; et
une unité de commande (100) reliée de façon opérationnelle à la pompe de la solution d'albumine (50) et au capteur de pression du circuit de la solution d'albumine (44), où l'unité de commande (100) est configurée pour actionner la pompe de la solution d'albumine (50) de sorte que la pression du circuit de la solution d'albumine reste dans un intervalle de valeurs sélectionné en variant un débit de la solution d'albumine dans le circuit de la solution d'albumine.

2. Appareil (1) selon la revendication 1, où l'unité de commande (100) est configurée pour commander le débit de la solution d'albumine en utilisant la pompe de la solution d'albumine (50) de façon indépendante d'un débit du sang fourni par la pompe du sang (20) dans le circuit du sang.

3. Appareil (1) selon l'une quelconque des revendications 1 à 2, où l'unité de commande (100) est configurée pour actionner la pompe de la solution d'albumine (50) à ou au-dessous d'un débit maximum sélectionné.

4. Appareil (1) selon l'une quelconque des revendications 1 à 3, où l'unité de commande (100) est configurée pour actionner la pompe de la solution d'albumine (50) à ou au-dessus d'un débit minimum sélectionné.

5. Appareil (1) selon l'une quelconque des revendications 1 à 4, où le capteur de pression du circuit de la solution d'albumine (44) est placé dans le circuit de la solution d'albumine en aval de la pompe de la solution d'albumine (50) et en amont du compartiment de chargement de la solution d'albumine (34) du filtre primaire (30).

6. Appareil (1) selon l'une quelconque des revendications 1 à 4, où le circuit de la solution d'albumine comprend un ou plusieurs filtres (62, 64, 66) placés entre la pompe de la solution d'albumine (50) et le compartiment de chargement de la solution d'albumine (34) du filtre primaire (30), où l'un ou plusieurs filtres (62, 64, 66) sont configurés pour éliminer du matériau sélectionné du fluide avant que le fluide n'entre dans le compartiment de chargement de la solution d'albumine (34) du filtre primaire (30), et où le capteur de pression du circuit de la solution d'albumine (44) est placé dans le circuit de la solution d'albumine en aval de la pompe de la solution d'albumine (50) et en amont de l'un ou plusieurs filtres.

7. Appareil (1) selon l'une quelconque des revendications 1 à 6, où la pompe de la solution d'albumine (50) est placée en aval du compartiment de rinçage de la solution d'albumine (72) du filtre du dialysat (70).

8. Appareil (1) selon l'une quelconque des revendications 1 à 6, où la pompe de la solution d'albumine (50) est placée en amont du compartiment de rinçage (72) de la solution d'albumine du filtre du dialysat (70).

9. Appareil (1) selon l'une quelconque des revendications 1 à 8, où des variations dans une pression du sang dans le compartiment du sang (32) du filtre primaire (30) changent la pression du circuit de la solution d'albumine.

10. Appareil (1) selon l'une quelconque des revendications 1 à 9, où des variations dans une pression du dialysat dans le compartiment du dialysat (74) du filtre du dialysat (70) changent la pression du circuit de la solution d'albumine.

11. Appareil (1) selon l'une quelconque des revendications 1 à 10, où l'intervalle de valeurs sélectionné pour la pression du circuit de la solution d'albumine comprend une valeur constante sélectionnée.
